# EUROPÄISCHE PATENTSCHRIFT

(11) **EP 0 480 006 B1**
(45) Veröffentlichungstag und Bekanntmachung des Hinweises auf die Patenterteilung: **05.08.1998**
(21) Anmeldenummer: 91908268.5
(22) Anmeldetag: 23.04.1991
(51) Int. Cl.: C07D 209/14

(54) **VERFAHREN ZUR HERSTELLUNG VON GRAMIN-DERIVATEN**
PROCESS FOR PRODUCING GRAMINE DERIVATES
PROCEDE DE PRODUCTION DE DERIVES DE GRAMINE

(30) Priorität: 26.04.1990 DE 4013907
(43) Veröffentlichungstag der Anmeldung: 15.04.1992
(73) Patentinhaber: SCHERING AKTIENGESELLSCHAFT, 13342 Berlin (DE)
(72) Erfinder: KÜBLER, Wolfgang, D-1000 Berlin 27 (DE); HAFFER, Gregor, D-1000 Berlin 45 (DE); WIERZCHOWSKI, Reiner, D-1000 Berlin 65 (DE); NICKISCH, Klaus, D-1000 Berlin 49 (DE)
(86) Internationale Anmeldenummer: DE9100353
(87) Internationale Veröffentlichungsnummer: WO9116304

(56) Entgegenhaltungen:
- EP-A- 54 507
- US-A- 3 459 767
- Journal of Heterocyclic Chemistry, Band 7, Nr. 1, Februar 1970, S. Raines et al.: "A Study on the Condensation of pyrroles, formaldehyde and primary amine hydrochlorides", Seiten 223-225

## Beschreibung

Die Erfindung betrifft ein Verfahren zur Herstellung von Gramin-Derivaten.

Gramine sind Zwischenprodukte für die Synthese pharmakologisch anwendbarer Verbindungen wie beispielsweise zur Herstellung von Tryptaminen (C.A. 56, 11701 (1962), Tryptophanen (R.V. Heinzelmann et al. Org. Chemie 25, 1548 (1960)) und Carbolinen (EP-A-239667).

Aufgrund ihrer guten Bindungsaffinität an die Benzodiazepin-Rezeptoren zeigen β-Carboline Wirkungen auf das Zentralnervensystem und haben daher in letzter Zeit großes Interesse in der Arzneimittelforschung gefunden. Während die Graminbildung mit Formaldehyd und sekundären Aminen oft problemlos abläuft, gelingt die Umsetzung des Indols mit Aldehyden und primären Aminen nur mit schlechten Ausbeuten (H.R. Snyder et al., J. Am. Soc. 79, 2217 (1957). Bei Verwendung der entsprechenden Imine ist die Ausbeute zwar besser, aber in Abhängigkeit von den Substituenten des Indols immer noch unbefriedigend. Beispielsweise wird die Umsetzung von Indolen mit Aldiminen in saurem Medium in U.S.P. 3.459,767 beschrieben.

Es stellte sich daher die Aufgabe ein Verfahren zu entwickeln, das auf Grund seiner guten Ausbeuten bei gleichzeitig guter Handhabung und ohne aufwendige Trennoperationen die großtechnische Herstellung dieser β-Carbolin-Zwischenstufen ermöglicht.

Uberraschenderweise wurde nun gefunden, daß die Umsetzung von Indol-Derivaten mit Iminen mit nahezu quantitativen Ausbeuten abläuft, wenn dem Reaktionsgemisch dem Imin entsprechende primäre Amine zugesetzt werden.

Da durch den Aminzusatzt weder ein zusätzlicher Reaktionsschritt erforderlich ist, noch schwer abtrennbare Nebenprodukte entstehen, ist dieses Verfahren sehr gut für die großtechnische Synthese dieser wichtigen Zwischenverbindung zur Herstellung wertvoller Pharmaka geeignet.

Die Erfindung betrifft das Verfahren zur Herstellung von Verbindungen der Formel I 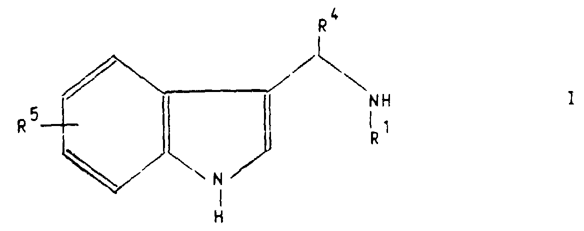 worin
- R¹: C₁₋₄-Alkyl,
- R⁴: C₁₋₄ Alkyl oder C₁₋₄ Alkoxy-C₁₋₂-Alkyl,
- R⁵: OR⁶ oder CHR⁷R⁸ ist, wobei
- R⁶: C₁₋₄-Alkyl C₃₋₆-Cycloalkyl oder ein gegebenenfalls substituierter Aralkylrest ist,
- R⁷: Wasserstoff oder C₁₋₄-Alkyl,
- R⁸: Wasserstoff, C₁₋₄-Alkyl, OR⁹ oder NR¹⁰R¹¹ mit R⁹ in der Bedeutung von C₁₋₄-Alkyl und R¹⁰ und R¹¹ gleich oder verschieden sind und Wasserstoff, C₁₋₄-Alkyl oder gemeinsam mit dem Stickstoffatom einen gegebenfalls ein weiteres Heteroatom enthaltenden gesättigten heterocyclischen Fünf- oder Sechsring bedeuten, der gegebenenfalls mit ein bis zwei C₁₋₄-Alkylgruppen substituiert ist, dadurch gekennzeichnet,
daß man ein Indolderivat-Derivat der Formel II 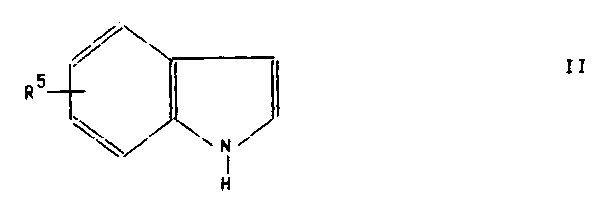 worin R⁵ die obige Bedeutung hat mit einem Imin der Formel III

R⁴-CH=N-R¹ III,

worin
R⁴ und R¹ die obige Bedeutung haben, unter Säurekatalyse in Gegenwart von primären Aminen umsetzt.

Der Substituent R⁵ kann in 4-, 5-, 6- und/oder 7-Stellung ein- oder zweifach stehen, wobei die Substitution in 4- oder 5-Stellung bevorzugt ist.

Unter C₁₋₄-Alkyl ist jeweils eine gerade oder verzweigte Alkylgruppe mit 1-4 Kohlenstoffatomen zu verstehen wie beispielsweise Methyl, Ethyl, Propyl, Isopropyl, Butyl, sek. Butyl, tert. Butyl und Isobutyl.

Geeignete Cycloalkylreste sind beispielsweise Cyclopropyl, Cyclobutyl, Cyclopentyl und Cyclohexyl.

Bedeuten R¹⁰ und R¹¹ gemeinsam mit dem Stickstoffatom einen gegebenenfalls ein weiteres Heteroatom wie Sauerstoff, Stickstoff oder Schwefel enthaltenden 5- oder 6-gliedrigen Heterocyclus, so stellt dieser beispielsweise Morpholin, Piperidin, Thiomorpholin, Piperazin oder Pyrrolidin dar und kann gegebenenfalls mit ein bis zwei C₁₋₄-Alkylgruppen substituiert sein wie beispielsweise 2,6-Dimethylmorpholin und N-Methyl-piperazin.

Der Aralkylrest R⁶ kann 7-8 Kohlenstoffatome enthalten und stellt bevorzugt den Phenyl-C₁₋₂-alkylrest dar wie beispielsweise Benzyl, Phenethyl und α-Methylbenzyl.

Als Substituenten des Aralkylrestes sind Halogene, wie Fluor, Chlor, Brom oder Jod, C₁₋₄-Alkoxy oder C₁₋₄-Alkyl geeignet, wobei der Substituent ein-oder zweifach in jeder Position des Arylrestes stehen kann. Als bevorzugter Aralkylrest ist der Benzylrest zu betrachten, der mit Halogen ein- bis 2-fach substituiert sein kann wie 3-Brombenzyl und 4-Chlorbenzyl.

Als bevorzugte Substituentenkombination für das erfindungsgemäße Verfahren ist R⁴ in der Bedeutung von C₁₋₄-Alkoxy-C₁₋₂-alkyl und R⁵ in der Bedeutung von OR⁶, mit R⁶ in der oben genannten Bedeutung zu betrachten.

Die erfindungsgemäße Umsetzung wird säurekatalysiert beispielsweise mit organischen Säuren wie Ameisensäure, Essigsäure, Propionsäure oder Gemischen der organischen Säuren durchgeführt. Das dem Reaktionsgemisch zugefügte primäre Amin entspricht vorteilhafterweise dem zur Imindarstellung verwendeten Amin R¹-NH₂, um die Aufarbeitung der Reaktion zu vereinfachen. Selbstverständlich sind auch andere aliphatische Amine geeignet.

Als Lösungsmittel können inerte Lösungsmittel wie Kohlenwasserstoffe, chlorierte Kohlenwasserstoffe oder cyclische oder acyclische Ether wie beispielsweise Toluol, Benzol, Xylol, Diethylether, Dimethoxymethan oder Methyl-tert.butylether zugefügt werden oder die Umsetzung wird in Suspension durchgeführt.

Das Verfahren wird im allgemeinen bei Temperaturen von - 20 °C bis zu Raumtemperatur durchgeführt. Das Imin wird in equivalenten Mengen oder im Uberschuß zugefügt.

Nach ca. 0,5 bis 24 Stunden ist die Reaktion beendet. Es wird in üblicher Weise aufgearbeitet und das Endprodukt durch Filtration und Kristallisation isoliert. Die so erhaltenen Verbindungen der Formel I können nach den beispielsweise in EP-54507 und EP-A-239667 beschriebenen Verfahren in sehr gut wirksame Pharmaka überführt werden.

Die Herstellung der Ausgangsverbindungen ist bekannt oder erfolgt nach bekannten Verfahren.

Die nachfolgenden Beispiele sollen das erfindungsgemäße Verfahren erläutern.

### Darstellung der Ausgangsverbindung

72,5 ml Isopropylamin werden in 115 ml Toluol unter Stickstoffatmosphäre gelöst und bei 0 °C mit 56,5 ml Methoxyacetaldehyd versetzt. Nach 30 Minuten werden 38 g Kaliumcarbonat zugegeben und anschließend filtriert. Der Rückstand wird mit Toluol gewaschen. Das Volumen der Lösung beträgt etwa 400 ml. Das ohne Toluol hergestellte Imin zeigt folgendes ¹H-NMR- Spektrum: 90 MHz (CDCl₃):
7,74 tr J=4 Hz (1); 4,04 d J=4 Hz (2); 3,70 s (3); 3,69 m (1); 1,18 d J=6 Hz (6)

### Beispiel 1

### Darstellung des 5-Benzyloxygraminderivates

Zu einer Suspension aus 95 g 5-Benzyloxyindol, 300 ml Toluol, 300 ml Eisessig, 200 ml Ameisensäure und 100 ml Isopropylamin werden bei - 10 °C 340 ml (1,6 eqv.) der Iminlösung zugetropft. Man läßt eine Stunde Rühren und gibt das Reaktionsgemisch zu 800 ml Wasser. Die wässrige Phase wird zweimal mit Toluol extrahiert und danach mit 50%iger Natronlauge und Kaliumcarbonat von pH 3 auf pH 11,5 -12,0 eingestellt. Der entstandene Niederschlag wird abfiltriert, mit Wasser gewaschen und getrocknet. Ausbeute: 137 g 5-Benyzloxypseudogramin = 95,2 % der Theorie, Schmelzpunkt: 109-111 °C.

### Beispiel 2

### Darstellung des 4-Benzyloxygraminderivates

Zu einer Lösung aus 9,4 g 4-Benzyloxyindol, 10 ml Toluol, 30 ml Eisessig, 20 ml Ameisensäure und 10 ml Isopropylamin werden bei - 10 °C 53,2 ml (2,5 eqv.) der Iminlösung zugetropft. Man läßt drei Stunden Rühren und gibt das Reaktionsgemisch zu 300 ml Wasser. Die wässrige Phase wird zweimal mit Toluol extrahiert und danach mit 50Ziger Natronlauge und Kaliumcarbonat von pH 3 auf pH 11,5 - 12,0 eingestellt. Der entstandene Niederschlag wird abfiltriert, mit Wasser gewaschen und getrocknet.

Ausbeute: 12,87 g 4-Benzyloxypseudogramin = 90,4 % der Theorie, Schmelzpunkt: 127-133 °C.

## Patentansprüche

1. Verfahren zur Herstellung von Verbindungen der Formel I 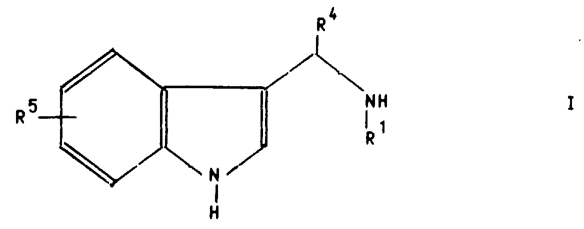 worin
R¹ C₁₋₄-Alkyl,
R⁴ C₁₋₄ Alkyl oder C₁₋₄ Alkoxy-C₁₋₂-Alkyl und
R⁵ OR⁶ oder CHR⁷R⁸ ist, wobei
R⁶ C₁₋₄-Alkyl, C₃₋₆-Cycloalkyl oder ein gegebenfalls substituierter Aralkylrest ist,
R⁷ Wasserstoff oder C₁₋₄-Alkyl und
R⁸ Wasserstoff, C₁₋₄-Alkyl, OR⁹ oder NR¹⁰R¹¹ mit R⁹ in der Bedeutung von C₁₋₄-Alkyl und R¹⁰ und R¹¹ gleich oder verschieden sind und Wasserstoff, C₁₋₄-Alkyl oder gemeinsam mit dem Stickstoffatom einen gegebenfalls ein weiteres Heteroatom enthaltenden gesättigten heterocyclischen Fünf- oder Sechsring bedeuten, der gegebenfalls mit ein bis zwei C₁₋₄-Alkylgruppen substituiert ist,
dadurch gekennzeichnet,
daß man ein Indolderivat-Derivat der Formel II 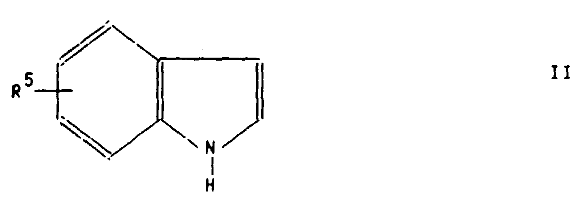 worin R⁵ die obige Bedeutung hat mit einem Imin der Formel III
R⁴-CH=N-R¹ III,
worin
R⁴ und R¹ die obige Bedeutung haben, unter Säurekatalyse in Gegenwart von primären Aminen umsetzt.

2. Verfahren nach Anspruch 1, dadurch gekennzeichnet, daß R¹ Isopropyl ist.

## Claims

1. Process for the preparation of compounds of formula I 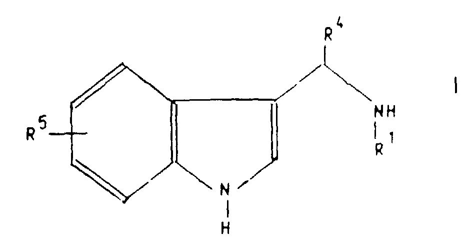 wherein
R¹ represents C₁₋₄alkyl,
R⁴ represents C₁₋₄alkyl or C₁₋₄alkoxy-C₁₋₂alkyl, and
R⁵ represents OR⁶ or CHR⁷R⁸, wherein
R⁶ is C₁₋₄alkyl, C₃₋₆cydoalkyl or an optionally substituted aralkyl radical,
R⁷ represents hydrogen or C₁₋₄alkyl, and
R⁸ represents hydrogen, C₁₋₄alkyl, OR⁹ or NR¹⁰R¹¹ wherein R⁹ is C₁₋₄alkyl and
R¹⁰ and R¹¹ are the same or different and represent hydrogen or C₁₋₄alkyl, or, together with the nitrogen atom, form a saturated heterocyclic 5- or 6-membered ring that optionally contains a further hetero atom and is optionally substituted by one or two C₁₋₄alkyl group(s),
characterised in that an indole derivative of formula II 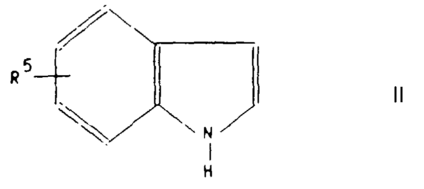 wherein R⁵ is as defined above is reacted with an imine of formula III
R⁴-CH=N-R¹ III,
wherein R⁴ and R¹ are as defined above, with acid catalysis and in the presence of primary amines.

2. Process according to claim 1, characterised in that R¹ is isopropyl.

## Revendications

1. Procédé de préparation des composés de formule I 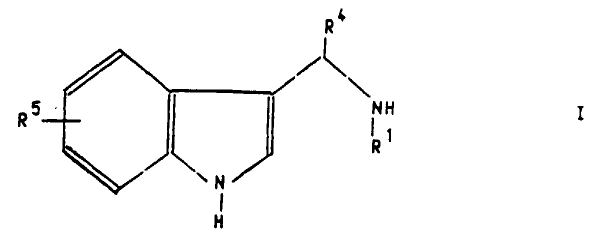 dans laquelle
R¹ représente un groupe alkyle en C₁₋₄,
R⁴ représente un groupe alkyle en C₁₋₄ ou alkoxy en C₁₋₄-alkyle en C₁₋₂,
R⁵ est OR⁶ ou CHR⁷R⁸, dans lesquelles
R⁶ est un radical alkyle en C₁₋₄, cycloalkyle en C₃₋₆ ou un radical aralkyle éventuellement substitué,
R⁷ représente un atome d'hydrogène ou un groupe alkyle en C₁₋₄,
R⁸ représente un atome d'hydrogène, un groupe alkyle en C₁₋₄, OR⁹ ou NR¹⁰R¹¹ avec R⁹ choisi parmi des groupes alkyle en C₁₋₄, et R¹⁰ et R¹¹ sont identiques ou différents et représentent un atome d'hydrogène, un groupe alkyle en C₁₋₄, ou ensemble avec l'atome d'azote un hétérocycle saturé à cinq ou six chaînons contenant éventuellement un autre hétéroatome, lequel hétérocycle est éventuellement substitué avec un à deux groupes alkyle en C₁₋₄,
caractérisé en ce que,
l'on fait réagir, sous catalyse acide en présence d'amines primaires, un dérivé d'un dérivé de l'indole de formule II 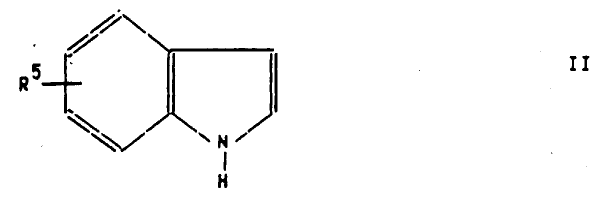 dans laquelle R⁵ a la signification ci-dessus avec une imine de formule III
R⁴ -CH = N- R¹ III,
dans laquelle
R⁴ et R¹ ont la signification ci-dessus.

2. Procédé selon la revendication 1, caractérisé en ce que R¹ est un groupe isopropyle.
